# EUROPÄISCHE PATENTSCHRIFT

(11) **EP 0 582 926 B1**
(45) Veröffentlichungstag und Bekanntmachung des Hinweises auf die Patenterteilung: **23.12.1998**
(21) Anmeldenummer: 93112328.5
(22) Anmeldetag: 31.07.1993
(51) Int. Cl.: C07D 241/08, A01N 43/60

(54) **Piperazin-2,3-dione als Pflanzenwachstumsregulatoren**
Piperazin-2,3-diones as plant growth regulators
Pipérazine-2,3-diones pour la régulation de croissance de plantes

(30) Priorität: 11.08.1992 DE 4226558
(43) Veröffentlichungstag der Anmeldung: 16.02.1994
(73) Patentinhaber: BASF Aktiengesellschaft, 67063 Ludwigshafen (DE)
(72) Erfinder: Rentzea, Costin, Dr., D-6900 Heidelberg (DE); Harreus, Albrecht, Dr., D-6700 Ludwigshafen (DE); Landes, Andreas, Dr., D-6703 Limburgerhof (DE); Walter, Helmut, Dr., D-6719 Obrigheim (DE); Rademacher, Wilhelm, Dr., D-6703 Limburgerhof (DE)

(56) Entgegenhaltungen:
- EP-A- 0 469 423
- US-A- 4 057 413
- ARCHIV DER PHARMAZIE Bd. 312, Nr. 12, 1979, Seiten 1019 - 1026 SCHMIDT J. & ZINNER G. '1,3-Dihydroxyharnstoffe, 2.Mitt.'
- CHEMICAL ABSTRACTS, vol. 111, no. 19, 6. November 1989, Columbus, Ohio, US; abstract no. 169382x, HUDEC J ET AL. 'N,N-bis(hydroxyethyl)piperazine plant growth stimulator' Seite 269 ;Spalte 2 ;
- CHEMICAL ABSTRACTS, vol. 72, no. 1, 5. Januar 1970, Columbus, Ohio, US; abstract no. 3053s, OST W ET AL. 'N,N'-Bis(1-alkanamido-2,2,2 trichloroethyl)piperazines and -alkylenediamines' Seite 270 ;Spalte 1 ;
- CHEMICAL ABSTRACTS, vol. 70, no. 5, 3. Februar 1969, Columbus, Ohio, US; abstract no. 20066z, OKADA M ET AL. 'Carbamates of N-hydroxy nitrogen heterocycles' Seite 1999 ;Spalte 2 ;

## Beschreibung

Die vorliegende Erfindung betrifft Piperazin-2,3-dione der Formel I 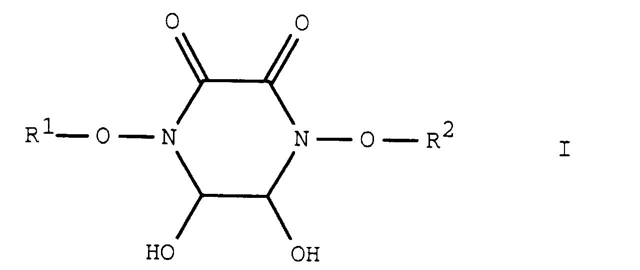 in der die Substituenten folgende Bedeutung haben:
R¹ und R² unabhängig voneinander
C₁-C₂₀-Alkyl, C₃-C₁₈-Alkenyl oder C₃-C₈-Alkinyl, wobei diese Gruppen ein bis fünf Halogenatome tragen können;
C₃-C₁₀-Cycloalkyl,C₅-C₁₀-Cycloalkenyl, C₃-C₁₀-Cycloalkylmethyl, C₅-C₁₀-Cycloalkenylmethyl, Norbornyl, Adamantyl oder Tricyclodecanyl, wobei die Ringe ein bis drei C₁-C₄-Alkylgruppen oder einen Phenylring tragen können;
Phenyl-C₁-C₄-alkyl oder Phenyl-C₂-C₆-alkenyl, wobei die aromatischen Reste ein bis fünf Halogenatome und/oder ein bis drei der folgenden Gruppen tragen können: Nitro, C₁-C₄-Alkyl, C₁-C₄-Halogenalkyl, C₁-C₄-Alkoxy, C₁-C₄-Halogenalkoxy, C₁-C₄-Alkylthio oder C₁-C₄-Halogen-alkylthio.

Außerdem betrifft die Erfindung Verfahren zur Herstellung dieser Verbindungen, sie enthaltende Mittel und Verfahren zu ihrer Verwendung als Pflanzenwachstumsregulatoren.

In EP-A 469 423 werden bereits Oxalyl-bishydroxamsäurederivate beschrieben, die pflanzenregulatorische Eigenschaften aufweisen. Aus Chemical Abstracts (1989) 111, 169328X,

US-A-4 057 413, Chemical Abstracts (1970) 72,3053s und Chemical Abstracts (1969) 70, 20066Z sind weiter Piperazin-Derivate bekannt, die ebenfalls als Wachstumsregulatoren verwendet werden können. In Archiv der Pharmazie (1979) 312, 1019-1026 werden weiter Piperazin-2,3-dion Derivate beschrieben, die als Cytostatika Verwendung finden können.

Aufgabe der vorliegenden Erfindung waren neue wirksame Pflanzenwachstumsregulatoren.

Demgemäß wurden die eingangs definierten Piperazin-2,3-dione I gefunden. Außerdem wurden Verfahren zur Herstellung dieser Verbindungen, sie enthaltende Mittel und Verfahren zu ihrer Verwendung als Pflanzenwachstumsregulatoren gefunden.

Man erhält die Piperazin-2,3-dione der Formel I beispielsweise dadurch, daß man einen Oxalyl-bishydroxamsäurederivat der Formel II, in an sich bekannter Weise in einem organischen Lösungsmittel oder in Wasser mit wasserfreiem Glyoxal oder mit dessen wäßriger Lösungen gegebenenfalls in Gegenwart einer anorganischen oder organischen Base und gegebenenfalls in Gegenwart eines Katalysators umsetzt.

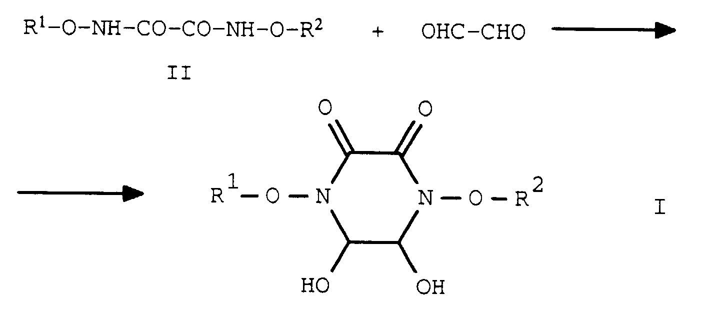

Die Umsetzung erfolgt im allgemeinen bei Temperaturen von 0 bis 100°C, vorzugsweise von 20 bis 80°C.

Als Lösungsmittel kommen beispielsweise Alkohole, wie Methanol, Ethanol, Propanol, Isopropanol und Butanol; Ether, wie Ethylenglykoldimethylether, Tetrahydrofuran und Dioxan; Nitrile wie Acetonitril und Propionitril; Amide, wie Dimethylformamid, Dimethylacetamid und N-Methylpyrrolidon; Säure wie Essigsäure und Propionsäure, gegebenenfalls auch Wasser und entsprechende Gemische in Betracht. Zweckmäßig verwendet man das Lösungsmittel in einer Menge von 100 bis 2000 Gew.-%, vorzugsweise von 200 bis 700 Gew.-% bezogen auf Ausgangsstoff II.

Die Herstellung der Oxalyl-bishydroxamsäurederivate II ist aus der Literatur bekannt (Chem. Ber., 27, 1111 (1894); DE-A 40 24 283).

Als Basen und/oder Katalysatoren kommen beispielsweise Kaliumhydroxid, Natriumhydroxid, Kaliumcarbonat, Natriumcarbonat, Lithiumhydroxid, Lithiumcarbonat, Natriumbicarbonat, Kaliumbicarbonat, Calciumhydroxid, Cycliumoxid, Bariumoxid, Magnesiumhydroxid, Magnesiumoxid, Bariumhydroxid, Calciumcarbonat, Magnesiumcarbonat, Magnesiumbicarbonat, Magnesiumacetat, Zinkhydroxid, Zinkoxid, Zinkcarbonat, Zinkbicarbonat, Zinkacetat, Natriumformiat, Natriumacetat, Trimethylamin, Triethylamin, Tripropylamin, Triisopropylamin, Tributylamin, Triisobutylamin, Tribenzylamin, Tricyclohexylamin, Triamylamin, Trihexylamin, N,N-Dimethyl-anilin, N,N-Diethylanilin,N,N-Dipropylanilin, N,N-Dimethyl-toluidin, N,N-Diethyltoluidin, N,N-Dipropyltoluidin, N,N-Dimethyl-4-aminopyridin, N,N-Diethyl-4- aminopyridin, N,N-Dipropyl-4-aminopyridin, N,N-Dipropyl-4-aminopyridin, N-Methylpiperidin, N-Ethylpiperidin, N-Methyl-pyrrolidin, N-Ethyl-pyrrolidin, N-Methylimidazol, N-Ethylimidazol, N-Methylmorpholin, N-Ethylmorpholin, N-Methylhexamethylenimin, N-Ethylhexamethylenimin, Pyridin, Chinolin, alpha-Picolin, beta-Picolin, Isochinolin, N,N,N',N'-Tetramethylethylen-diamin, N,N,N',N'-Tetraethylethylendiamin, N-Propyldiisopropylamin, N,N-Dimethylcyclohexylamin, 2,6-Lutidin, 2,4-Lutidin, Triethylendiamin in Betracht.

Zweckmäßig verwendet man die Base in Mengen von jeweils 1 bis zu 20 Mol.-%, bezogen auf den Ausgangsstoff II.

Im Hinblick auf die bestimmungsgemäße Verwendung der Verbindungen I und sie enthaltende wachstumsregulierende Mittel kommen als Substituenten folgende Reste in Betracht:
R¹ und R² unabhängig voneinander
C₁-C₂₀-Alkyl, vorzugsweise verzweigtes oder unverzweigtes C₁-C₆-Alkyl wie Methyl, Ethyl, Propyl, 1-Methylethyl, Butyl, 1-Methyl-propyl, 2-Methyl-propyl, 1,1-Dimethylethyl, Pentyl, 1-Methylbutyl, 2-Methylbutyl, 3-Methylbutyl, 2,2-Dimethylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethyl-butyl, 2-Ethylbutyl, 1,1,2-Trimethylpropyl, 1,2,2-Trimethylpropyl, 1-Ethyl-1-methylpropyl und l-Ethyl-2-methylpropyl, insbesondere verzweigtes oder unverzweigtes C₁-C₄-Alkyl wie Methyl, Ethyl, Propyl, 1-Methylethyl, Butyl, 1-Methyl-propyl, 2-Methylpropyl und 1,1-Dimethylethyl;
C₃-C₁₈-Alkenyl, besonders C₃-C₆-Alkenyl wie 2-Propenyl, 2-Butenyl, 3-Butenyl, 1-Methyl-2-propenyl, 2-Methyl-2-propenyl, 2-Pentenyl, 3-Pentenyl, 4-Pentenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-2-propenyl, 1-Ethyl-2-propenyl, 2-Hexenyl, 3-Hexenyl, 4-Hexenyl, 5-Hexenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-2-butenyl, 1,2-Dimethyl-3-butenyl, 1,3-Dimethyl-2-butenyl, 1,3-Dimethyl-3-butenyl, 2,2-Dimethyl-3-butenyl, 2,3-Dimethyl-2-butenyl, 2,3-Dimethyl-3-butenyl, 3,3-Dimethyl-2-butenyl, 1-Ethyl-2-butenyl, 1-Ethyl-3-butenyl, 2-Ethyl-2-butenyl, 2-Ethyl-3-butenyl, 1,1,2-Trimethyl-2-propenyl, 1-Ethyl-1-methyl-2-propenyl und l-Ethyl-2-methyl-2-propenyl; oder
C₃-C₈-Alkinyl, besonders C₃-C₆-Alkinyl wie 2-Propinyl, 2-Butinyl, 3-Butinyl, 1-Methyl-2-propinyl, 2-Pentinyl, 3-Pentinyl, 4-Pentinyl, 1-Methyl-2-butinyl, 1-Methyl-3-butinyl, 2-Methyl-3-butinyl, 1,1-Dimethyl-2-propinyl, 1-Ethyl-2-propinyl, 2-Hexinyl, 3-Hexinyl, 4-Hexinyl, 5-Hexinyl, 1-Methyl-2-pentinyl, 1-Methyl-3-pentinyl, 1-Methyl-2-pentenyl, 2-Methyl-3-pentinyl, 2-Methyl-4-pentinyl, 3-Methyl-4-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 1-Ethyl-2-butinyl, 1-Ethyl-3-butinyl, 2-Ethyl-3-butinyl und 1-Ethyl-1-methyl-2-propinyl, vorzugsweise 2-Propinyl und 2-Butinyl;
wobei diese Gruppen ein bis fünf Halogenatome wie Fluor, Chlor, Brom und Jod, vorzugsweise Fluor oder Chlor tragen können;
C₃-C₁₀-Cycloalkyl wie Cyclopropyl, Cyclobutyl, Cyclopentyl, Cyclohexyl, Cycloheptyl, Cyclooctyl, Menthyl, vorzugsweise Cyclopropyl und Cyclohexyl, oder Norbornyl, Adamantyl, Tri-cyclodecanyl;
C₅-C₁₀-Cycloalkenyl wie vorzugsweise 2-Cyclohexen-1-yl;
C₃-C₁₀-Cycloalkylmethyl wie Cyclopropylmethyl, Cyclobutylmethyl, Cyclopentylmethyl, Cyclohexylmethyl, Cycloheptylmethyl, Cyclooctylmethyl, vorzugsweise Cyclopropylmethyl oder Cyclohexylmethyl;
C₅-C₁₀-Cycloalkenylmethyl wie vorzugsweise 1-Cyclohexenylmethyl, 2-Cyclohexenylmethyl und 3-Cyclohexenylmethyl;
wobei die Ringe ein bis drei C₁-C₄-Alkylgruppen wie Methyl, Ethyl, Propyl, 1-Methylethyl, Butyl, 1-Methylpropyl, 2-Methylpropyl und 1,1-Dimethylethyl, vorzugsweise Methyl und Ethyl, insbesondere Methyl;
oder einen Phenylring tragen können;
Phenyl-C₁-C₄-alkyl wie Benzyl, 1-Phenylethyl, 2-Phenylethyl, 1-Phenylpropyl, 2-Phenylpropyl, 3-Phenylpropyl, 1-Methyl-1-phenylethyl, 1-Methyl-1-phenylethyl, 1-Phenylbutyl, 2-Phenylbutyl, 3-Phenylbutyl, 4-Phenylbutyl, 1-Methyl-1-phenylpropyl, 1-Methyl-2-phenylpropyl, 1-Methyl-3-phenylpropyl, 2-Methyl-1-phenylpropyl, 2-Methyl-2-phenylpropyl, 2-Methyl-3-phenylpropyl und 1,1-Dimethyl-2-phenylethyl, vorzugsweise Benzyl und 2-Phenylethyl;
oder
Phenyl-C₂-C₆-alkenyl, besonders Phenyl-C₂-C₄-alkenyl wie 2-Phenylethenyl, 2-Phenyl-1-propenyl, 2-Phenyl-2-propenyl, 2-Phenyl-1-methylethenyl, 2-Phenyl-1-butenyl, 2-Phenyl-2-butenyl, 2-Phenyl-3-butenyl, 2-Phenyl-1-methyl-1-propenyl und 2-Phenyl-1-methyl-2-propenyl;
wobei die aromatischen Reste ein bis fünf Halogenatome wie Fluor, Chlor, Brom und Jod, vorzugsweise Fluor, Chlor und Brom, insbesondere Fluor und Chlor und/oder ein bis drei der folgenden Gruppen tragen können: Nitro, C₁-C₄-Alkyl wie Methyl, Ethyl, Propyl, 1-Methylethyl, Butyl, 1-Methyl-propyl, 2-Methylpropyl und 1,1-Dimethylethyl, vorzugsweise Methyl, 1-Methylethyl und 1,1-Dimethylethyl; C₁-C₄-Halogenalkyl, besonders C₁-C₂-Halogenalkyl wie Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl, vorzugsweise Trifluormethyl; C₁-C₄-Alkoxy wie Methoxy, Ethoxy, Propyloxy, 1-Methylethoxy, Butyloxy, 1-Methylpropyloxy, 2-Methylpropyloxy und 1,1-Dimethylethoxy, vorzugsweise Methoxy und Ethoxy; C₁-C₄-Halogenalkoxy, besonders C₁-C₂-Halogenalkoxy wie Chlormethyloxy, Dichlormethyloxy, Trichlormethyloxy, Chlorfluormethyloxy, Dichlorfluormethyloxy, Chlordifluormethyloxy, 1-Fluorethyloxy, 2-Fluorethyloxy, 2,2-Difluorethyloxy, 2,2,2-Trifluorethyloxy, 2-Chlor-2-fluorethyloxy, 2-Chlor-2,2-difluorethyloxy, 2,2-Dichlor-2-fluorethyloxy, 2,2,2-Trichlorethyloxy und Pentafluorethyloxy; C₁-C₄-Alkylthio wie Methlythio, Ethylthio, Propylthio, 1-Methylethylthio, Butylthio, 1-Methylpropylthio, 2-Methylpropylthio und 1,1-Dimethylethylthio, vorzugsweise Methylthio; und C₁-C₄-Halogenalkylthio, besonders C₁-C₂-Halogenalkylthio wie Chlormethylthio, Dichlormethylthio, Trichlormethylthio, Chlorfluormethylthio, Dichlorfluormethylthio, Chlordifluormethylthio, 1-Fluorethylthio, 2-Fluorethylthio, 2,2-Difluorethylthio, 2,2,2-Trifluorethylthio, 2-Chlor-2-fluorethlythio, 2-Chlor-2,2-difluorethylthio, 2,2-Dichlor-2-fluorethylthio, 2,2,2-Trichlorethylthio und Pentafluorethylthio.

Im Hinblick auf die bestimmungsgemäße Verwendung in wachstumsregulierenden Mitteln sind Verbindungen I besonders bevorzugt, in denen R¹ folgende Bedeutung hat:

Methyl, Ethyl, Propyl, 1-Methylethyl, Butyl, 1-Methylpropyl, 2-Methylpropyl, 1,1-Dimethylethyl, Pentyl, 1-Methylbutyl, 2-Methylbutyl, 3-Methylbutyl, 2,2-Dimethylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und l-Ethyl-2-methylpropyl, insbesondere Methyl, Ethyl, Propyl, 1-Methylethyl, Butyl, 1-Methylpropyl, 2-Methlypropyl und 1,1-Dimethylethyl, 2-Propenyl, 2-Butenyl, 3-Butenyl, 1-Methyl-2-propenyl, 2-Methyl-2-propenyl, 3-Methyl-2-butenyl oder Benzyl, wobei diese Gruppen ein bis fünf Halogenatome wie Fluor, Chlor, Brom und Jod, vorzugsweise Fluor und Chlor tragen können.

Beispiele für insbesondere bevorzugte Piperazin-2,3-dione der Formel I sind in der folgenden Tabelle aufgeführt.

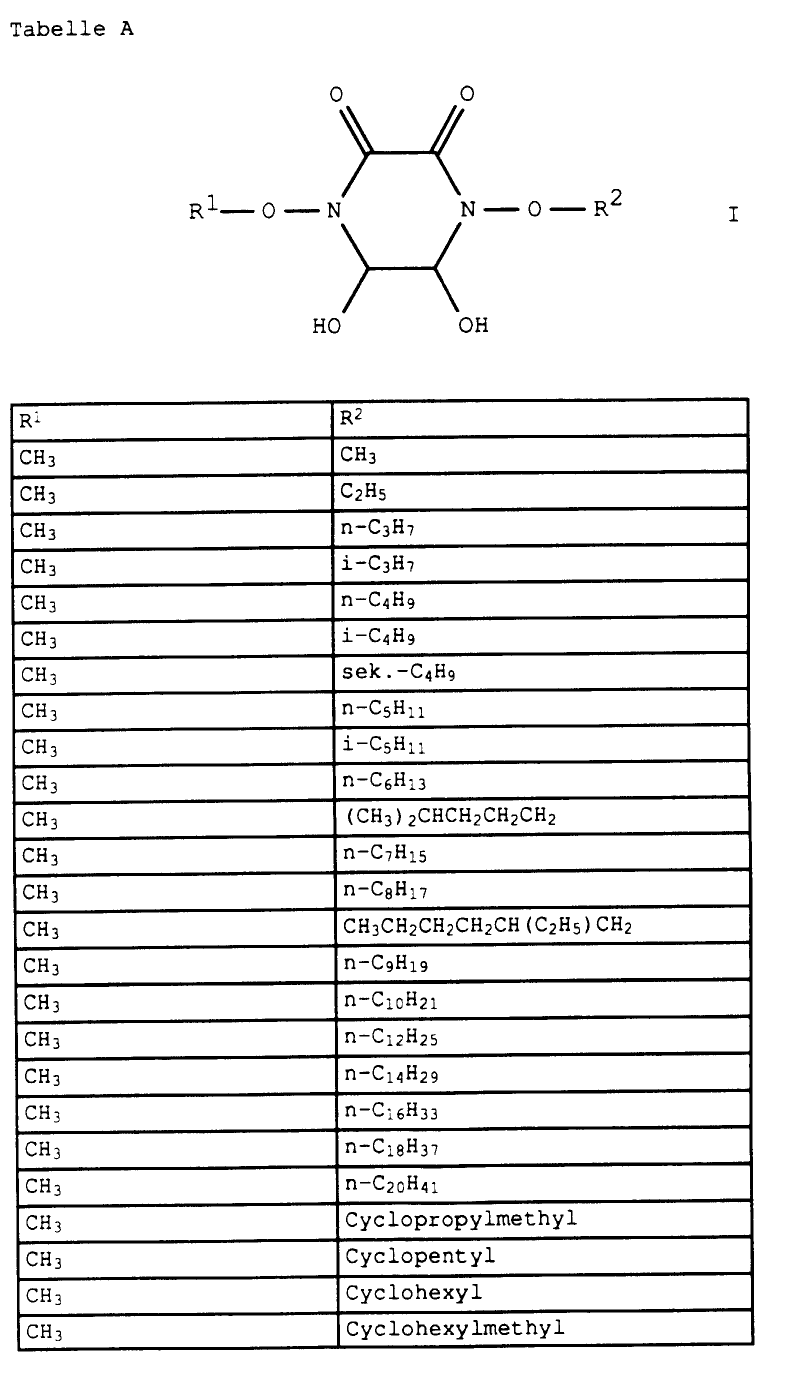

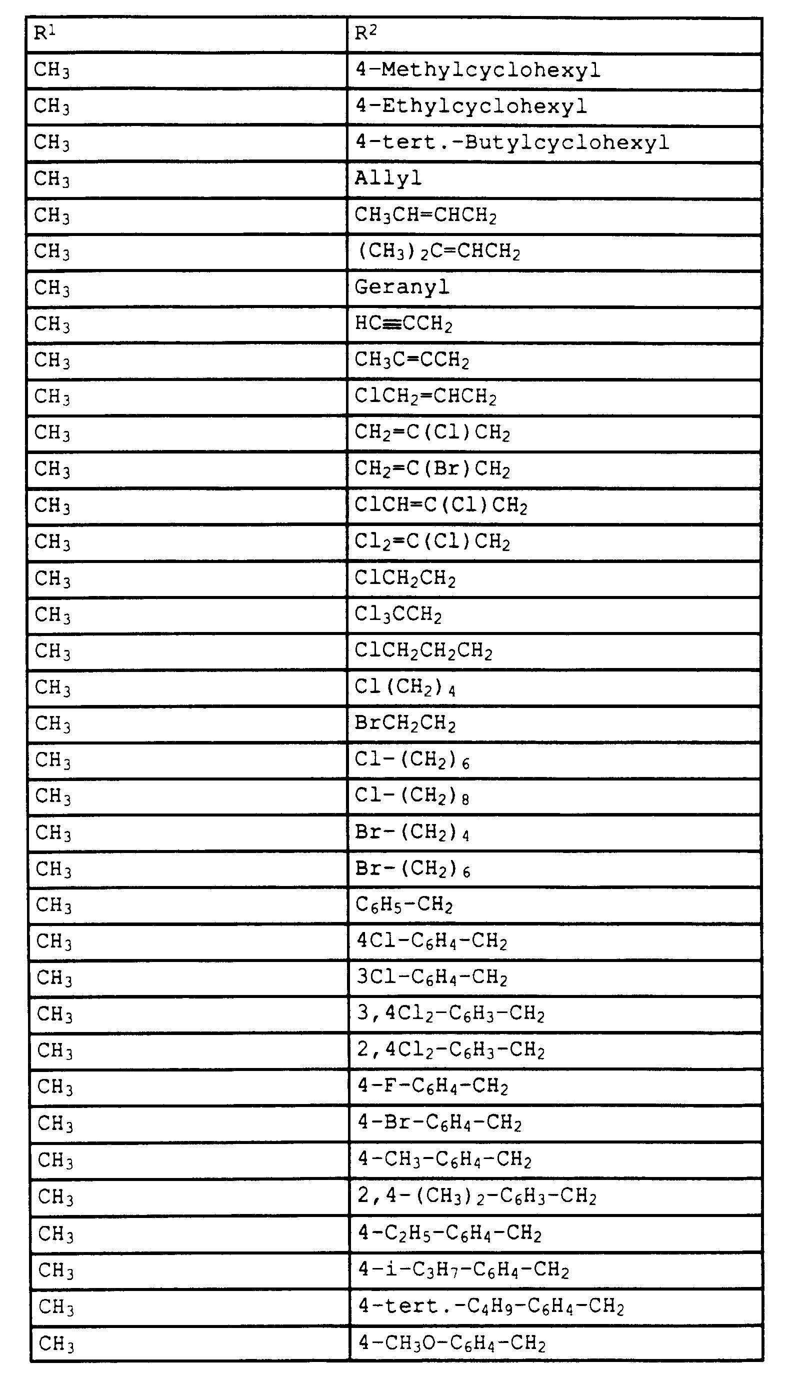

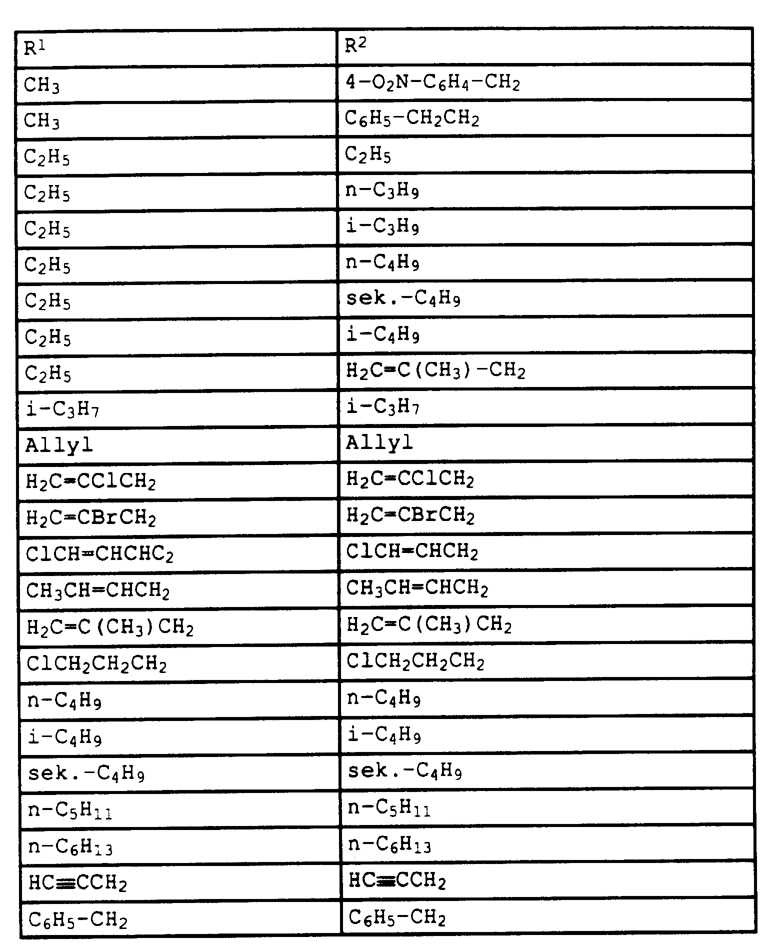

Die erfindungsgemäßen wachstumsregulierenden Wirkstoffe I bzw. die sie enthaltenden Mittel können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich nach den Verwendungszwecken; sie sollten in jedem Fall möglichst die feinste Verteilung der erfindungsgemäßen Wirkstoffe gewährleisten.

Die Wirkstoffe I eignen sich allgemein zur Herstellung von direkt versprühbaren Lösungen, Emulsionen, Pasten oder Öldispersionen. Als inerte Zusatzstoffe kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Toluol, Xylol, Paraffin, Tetrahydronaphthalin, alkylierte Naphthaline oder deren Derivate, Methanol, Ethanol, Propanol, Butanol, Cyclohexanol, Cyclohexanon, Chlorbenzol, Isophoron oder stark polare Lösungsmittel wie N,N-Dimethylformid, Dimethylsulfoxid, N-Methylpyrrolidon oder Wasser in Betracht.

Wäßrige Anwendungsformen können aus Emulsionskonzentraten, Dispersionen, Pasten, netzbaren Pulvern oder wassdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier-oder Emulgiermittel und eventuell Lösungsmittel oder Öl bestehende Konzentrate hergestellt werden, die zur Verdünnung mit Wasser geeignet sind.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether-und Fettalkoholsulfaten, sowie Salze sulfatierter Hexa-, Hepta-und Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenol-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylen, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Silicagel, Kieselsäuren, Kieselgele, Silikate, Talkum, Kaolin, Kalkstein, Kalk, Kreide, Bolus, Löß, Ton, Dolomit, Diatomeenerde, Calcium-und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe.

Die Formulierungen enthalten zwischen 0,1 und 95 Gew.-%, vorzugsweise zwischen 0,5 und 90 Gew.-%, Wirkstoff. Die Wirkstoffe werden dabei in einer Reinheit von 90 % bis 100 %, vorzugsweise 95% bis 100 % (nach NMR-Spektrum) eingesetzt.

Die erfindungsgemäßen Wirkstoffe I können beispielsweise wie folgt formuliert werden:
I. Man vermischt 90 Gewichtsteile der Verbindung Nr. 1 mit 10 Gewichtsteilen N-Methyl-α-pyrrolidon und erhält eine Lösung, die zur Anwendung in Form kleinster Tropfen geeignet ist.
II. 20 Gewichtsteile der Verbindung Nr. 2 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht.
   Durch Ausgießen und feines Verteilen der Lösung in 100000 Gewichtsteilen Wasser erhält man eine wäßrige Dispersion, die 0,02 Gew.-% des Wirkstoffs enthält.
III. 20 Gewichtsteile der Verbindung Nr. 2 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.-% des Wirkstoffs enthält.
IV. 20 Gewichtsteile des Wirkstoffs Nr. 2 werden in einer Mischung gelöst, die aus 25 Gewichtsteilen Cyclohexanon, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.-% des Wirkstoffs enthält.
V. 20 Gewichtsteile des Wirkstoffs Nr. 1 werden mit 3 Gewichtsteilen des Natriumsalzes der Diisobutylnaphthalin-α-sulfonsäure, 17 Gewichtsteilen des Natriumsalzes einer Ligninsulfonsäure aus einer Sulfit-Ablauge und 60 Gewichtsteilen pulverförmigen Kieselsäuregel gut vermischt und in einer Hammermühle vermahlen. Durch feines Verteilen der Mischung in 20000 Gewichtsteilen Wasser erhält man eine Spritzbrühe, die 0,1 Gew.-% des Wirkstoffs enthält.
VI. 3 Gewichtsteile des Wirkstoffs Nr. 3 werden mit 97 Gewichtsteilen feinteiligem Kaolin vermischt. Man erhält auf diese Weise ein Stäubemittel, das 3 Gew.-% des Wirkstoffs enthält.
VII. 30 Gewichtsteile des Wirkstoffs Nr. 3 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.
VIII. 20 Gewichtsteile des Wirkstoffs Nr. 4 werden mit 2 Gewichtsteilen Calciumsalz der Dodecylbenzolsulfonsäure, 8 Gewichtsteilen Fettalkohol-polyglykolether, 2 Gewichtsteilen Natriumsalz eines Phenol-Harnstoff-Formaldehyd-Kondensates und 68 Gewichtsteilen eines paraffinischen Mineralöls innig vermischt. Man erhält eine stabile ölige Dispersion.
IX. Man vermischt 90 Gewichtsteile der Verbindung Nr. 4 mit 10 Gewichtsteilen N-Methyl-α-pyrrolidon und erhält eine Lösung, die zur Anwendung in Form kleinster Tropfen geeignet ist.
X. 20 Gewichtsteile der Verbindung Nr. 4 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100000 Gewichtsteilen Wasser erhält man eine wäßrige Dispersion, die 0,02 Gew.-% des Wirkstoffs enthält.
XI. 20 Gewichtsteile der Verbindung Nr. 6 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethyletnoxid an 1 Mol Ricinusöl besteht. Durch Eingießen und feines Verteilen der Lösung in 100 000 Gewichtsteilen Wasser erhält man eine wäßrige Dispersion, die 0,02 Gew.-% des Wirkstoffs enthält.
XII. 20 Gewichtsteile des Wirkstoffs Nr. 7 werden in einer Mischung gelöst, die aus 25 Gewichtsteilen Cyclohexanol, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man einer wäßrigen Dispersion, die 0,02 Gew.-% des Wirkstoffs enthält.
XIII. 20 Gewichtsteile des Wirkstoffs Nr. 6 werden mit 3 Gewichtsteilen des Natriumsalzes der Diisobutylnaphthalin-α-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20000 Gewichtsteilen Wasser erhält man eine Spritzbrühe, die 0,1 Gew.-% des Wirkstoffs enthält.
XIV. 3 Gewichtsteile des Wirkstoffs Nr. 5 werden mit 97 Gewichtsteilen feinteiligem Kaolin vermischt. Man erhält auf diese Weise ein Stäubemittel, das 3 Gew.-% des Wirkstoffs enthält.
XV. 30 Gewichtsteile des Wirkstoffs Nr. 6 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit. XVI. 20 Gewichtsteile des Wirkstoffs Nr. 6 werden mit 2 Gewichtsteilen Calciumsalz der Dodecylbenzolsulfonsäure, 8 Gewichtsteilen Fettalkohol-polyglykolether, 2 Gewichtsteilen Natriumsalz eines Phenolsulfonsäure-Formaldehyd-Kondensates und 68 Gewichtsteilen eines paraffinischen Mineralöls innig vermischt. Man erhält eine stabile ölige Dispersion.

Die Aufwandmengen an Wirkstoff betragen je nach Jahreszeit, Zielpflanzen und Wachstumsstadium 0,001 bis 10, vorzugsweise 0,01 bis 5 kg/ha, insbesondere 0,05 bis 2 kg/ha aktive Substanz (a. S.).

Die Verbindungen der Formel IA können praktisch alle Entwicklungsstadien einer Pflanze verschiedenartig beeinflussen und werden deshalb als Wachstumsregulatoren eingesetzt. Die Wirkungsvielfalt der Pflanzenwachstumsregulatoren hängt vor allem
a) von der Pflanzenart und -sorte,
b) von dem Zeitpunkt der Applikation, bezogen auf das Entwicklungsstadium der Pflanze und von der Jahreszeit,
c) von dem Applikationsort und -verfahren z.B. (Samenbeize, Bodenbehandlung, Blattapplikation oder Stamminjektion bei Bäumen)
d) von klimatischen Faktoren, z.B. Temperatur, Niederschlagsmenge, außerdem auch Tageslänge und Lichtintensität
e) von der Bodenbeschaffenheit (einschließlich Düngung),
f) von der Formulierung bzw. Anwendungsform des Wirkstoffs und schließlich
g) von den angewendeten Konzentrationen der aktiven Substanz.

Aus der Reihe der verschiedenartigen Anwendungsmöglichkeiten der erfindungsgemäßen Pflanzenwachstumsregulatoren der Formel I im Pflanzenanbau, in der Landwirtschaft und im Gartenbau, werden einige nachstehend erwähnt.
A. Mit den erfindungsgemäß verwendbaren Verbindungen läßt sich das vegetative Wachstum der Pflanzen stark hemmen, was sich insbesondere in einer Reduzierung des Längenwachstums äußert. Die behandelten Pflanzen weisen demgemäß einen gedrungenen Wuchs auf; außerdem ist eine dunklere Blattfärbung zu beobachten.
   Als vorteilhaft für die Praxis erweist eine verminderte Intensität des Wachstums von Gräsern an Straßenrändern, Hecken, Kanalböschungen und auf Rasenflächen wie Park-, Sport- und Obstanlagen, Zierrasen und Flugplätzen, so daß der arbeits- und kostenaufwendige Rasenschnitt reduziert werden kann.
   Von wirtschaftlichem Interesse ist auch die Erhöhung der Standfestigkeit von lageranfälligen Kulturen wie Getreide, Mais, Sonnenblumen und Soja. Die dabei verursachte Halmverkürzung und Halmverstärkung verringern oder beseitigen die Gefahr des "Lagerns" (des Umknickens) von Pflanzen unter ungünstigen Witterungsbedingungen vor der Ernte.
   Wichtig ist auch die Anwendung von Wachstumsregulatoren zur Hemmung des Längenwachstums und zur zeitlichen Veränderung des Reifeverlaufs bei Baumwolle. Damit wird ein vollständig mechanisiertes Beernten dieser wichtigen Kulturpflanze ermöglicht.
   Bei Obst- und anderen Bäumen lassen sich mit den Wachstumsregulatoren Schnittkosten einsparen. Außerdem kann die Alternanz von Obstbäumen durch Wachstumsregulatoren gebrochen werden.
   Durch Anwendung von Wachstumsregulatoren kann auch die seitliche Verzweigung der Pflanzen vermehrt oder gehemmt werden. Daran besteht Interesse, wenn z.B. bei Tabakpflanzen die Ausbildung von Seitentrieben (Geiztrieben) zugunsten des Blattwachstums gehemmt werden soll.
   Mit Wachstumsregulatoren läßt sich beispielsweise bei Winterraps auch die Frostresistenz erheblich erhöhen. Dabei werden einerseits das Längenwachstum und die Entwicklung einer zu üppigen (und dadurch besonders frostanfälligen) Blatt- bzw. Pflanzenmasse gehemmt. Andererseits werden die jungen Rapspflanzen nach der Aussaat und vor dem Einsetzen der Winterfröste trotz günstiger Wachstumsbedingungen im vegetativen Entwicklungsstadium zurückgehalten. Dadurch wird auch die Frostgefährdung solcher Pflanzen beseitigt, die zum vorzeitigen Abbau der Blühhemmung und zum Übergang in die generativen Phase neigen. Auch bei anderen Kulturen, z.B. Wintergetreide ist es vorteilhaft, wenn die Bestände durch Behandlung mit erfindungsgemäßen Verbindungen im Herbst zwar gut bestockt werden, aber nicht zu üppig in den Winter hineingehen. Dadurch kann der erhöhten Frostempfindlichkeit und - wegen der relativ geringen Blatt- bzw. Pflanzenmasse - dem Befall mit verschiedenen Krankheiten (z.B. Pilzkrankheit) vorgebeugt werden. Die Hemmung des vegetativen Wachstums ermöglicht außerdem bei vielen Kulturpflanzen eine dichtere Bepflanzung des Bodens, so daß ein Mehrertrag bezogen auf die Bodenfläche erzielt werden kann.
   Mit den Wachstumsregulatoren lassen sich Mehrerträge sowohl an Pflanzenteilen als auch an Pflanzeninhaltsstoffen erzielen. So ist es beispielsweise möglich, das Wachstum größerer Mengen an Knospen, Blüten, Blättern, Früchten, Samenkörnern, Wurzeln und Knollen zu induzieren, den Gehalt an Zucker in Zuckerrüben, Zuckerrohr sowie Zitrusfrüchten zu erhöhen, den Proteingehalt in Getreide oder Soja zu steigern oder Gummibäume zum vermehrten Latexfluß zu stimulieren.
   Dabei können die Verbindungen der Formel IA Ertragssteigerungen durch Eingriffe in den pflanzlichen Stoffwechsel bzw. durch Förderung oder Hemmung des vegetativen und/oder des generativen Wachstums verursachen.
   Mit Pflanzenwachstumsregulatoren lassen sich schließlich sowohl eine Verkürzung bzw. Verlängerung der Entwicklungsstadien als auch eine Beschleunigung bzw. Verzögerung der Reife der geernteten Pflanzenteile vor oder nach der Ernte erreichen.
   Von wirtschaftlichem Interesse ist beispielsweise die Ernteerleichterung, die durch das zeitlich konzentrierte Abfallen oder Vermindern der Haftfestigkeit am Baum bei Zitrusfrüchten, Oliven oder bei anderen Arten und Sorten von Kern-, Stein- und Schalenobst ermöglicht wird. Derselbe Mechanismus, das heißt die Förderung der Ausbildung von Trenngewebe zwischen Frucht- bzw. Blatt- und Sproßteil der Pflanze ist auch für ein gut kontrollierbares Entblättern von Nutzpflanzen wie beispielsweise Baumwolle wesentlich.
D. Mit Wachstumsregulatoren kann weiterhin der Wasserverbrauch von Pflanzen reduziert werden. Dies ist besonders wichtig für landwirtschaftliche Nutzflächen, die unter einem hohen Kostenaufwand künstlich bewässert werden müssen, z.B. in ariden oder semiariden Gebieten. Durch den Einsatz der erfindungsgemäßen Substanzen läßt sich die Intensität der Bewässerung reduzieren und damit eine kostengünstigere Bewirtschaftung durchführen. Unter dem Einfluß von Wachstumsregulatoren kommt es zu einer besseren Ausnutzung des vorhandenen Wassers, weil u. a.
   - die Öffnungsweite der Stomata reduziert wird
   - eine dickere Epidermis und Cuticula ausgebildet werden
   - die Durchwurzelung des Bodens verbessert wird und
   - das Mikroklima im Pflanzenbestand durch einen kompakteren Wuchs günstig beeinflußt wird.

Die erfindungsgemäß zu verwendenden Wachstumsregulatoren der Formel I können den Kulturpflanzen sowohl vom Samen her (als Saatgutbeizmittel) als auch über den Boden, d.h. durch die Wurzel sowie - besonders bevorzugt - durch Spritzung über das Blatt zugeführt werden.

In Anbetracht der Vielseitigkeit der Applikationsmethoden können die erfindungsgemäßen Verbindungen bzw. sie enthaltende Mittel in einer großen Zahl von Kulturpflanzen eingesetzt werden.

Zur Verbreiterung des Wirkungsspektrums und zur Erzielung synergistischer Effekte können die erfindungsgemäßen Wirkstoffe IA mit zahlreichen Vertretern wachstumsregulierender Wirkstoffgruppen gemischt und gemeinsam ausgebracht werden.

Außerdem kann es von Nutzen sein, die Wirkstoffe I in Kombination mit Herbiziden und/oder mit Mitteln zur Bekämpfung von Schädlingen oder phytopathogenen Pilzen bzw. Bakterien gemeinsam auszubringen. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

### Synthesebeispiele

Die in den nachstehenden Synthesebeispielen wiedergegebenen Vorschriften wurden unter entsprechender Abwandlung der Ausgangsverbindung zur Gewinnung weiterer Wirkstoffe I benutzt. Die so erhaltenen Verbindungen sind in der nachstehenden Tabelle mit physikalischen Angaben aufgeführt.

### Beispiel 1

### 1,4-Bis(1-propenyl-3-oxy)-2,3-dioxo-5,6-dihydroxypiperazin

Eine Lösung von 30 g (0,15 Mol) Bis(1-propenyl-3-oxyamino)ethandion in 170ml Ethanol wurde mit 52,1 ml (0,36 Mol) einer 40%igen, wäßrigen Glyoxallösung versetzt und mit 1 N Natronlauge auf pH 7 bis 7,5 gebracht. Nach 72 h Rühren bei 25°C wurde das Gemisch bei vermindertem Druck eingeengt. Der Rückstand wurde mit 200 ml Essigester 30 Minuten gerührt, abfiltriert und das Filtrat bei vermindertem Druck von Lösungsmittel befreit. Der erhaltene Rückstand wurde mit 20 ml Ether und 0,5 ml Methanol bei 0°C zur Kristallisation gebracht. Man erhielt 12,1 g (31,2 % d. Theorie) 1,4-Bis(1-propenyl-3-oxy)-2,3-dioxo-5,6-dihydroxypiperazin als weiße Kristalle vom Schmp. 142 - 144°C.

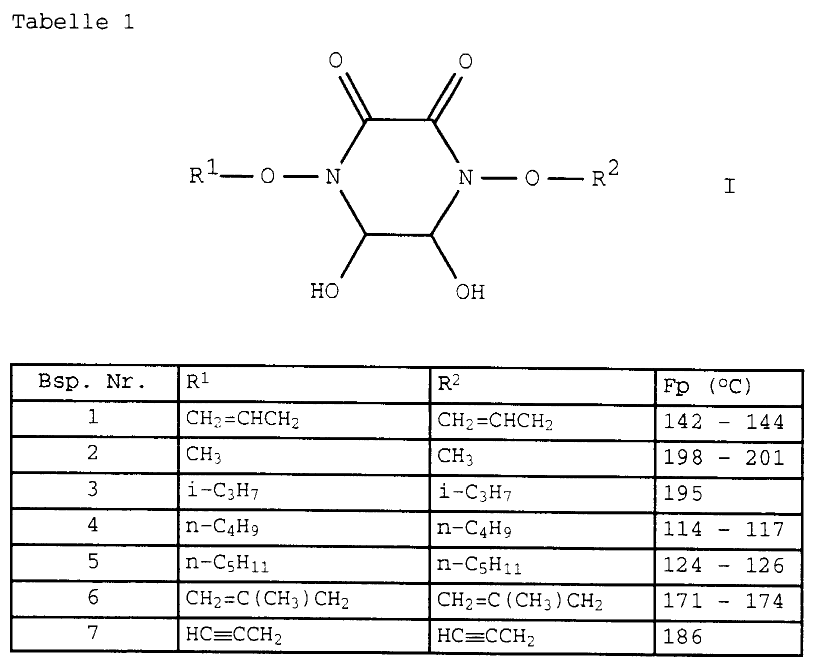

### Anwendungsbeispiele

Zur Bestimmung der wachstumsregulierenden Eigenschaften der Prüfsubstanzen wurden Testpflanzen auf ausreichend mit Nährstoffen versorgtem Kultursubstrat in Kunststoffgefäßen (ca. 8 cm Durchmesser; Volumen ca. 300 ml) angezogen.

Im Nachauflaufverfahren wurden die zu prüfenden Substanzen in wäßriger Aufbereitung auf die Pflanzen gesprüht. Die beobachtete wachstumsregulierende Wirkung wurde bei Versuchsende durch Wuchshöhenmessung belegt. Die so gewonnenen Meßwerte wurden zur Wuchshöhe der unbehandelten Pflanzen in Relation gesetzt.

Gleichlaufend zur Reduzierung des Längenwachstums stieg die Farbintensität der Blätter an. Der erhöhte Chlorophyllgehalt läßt eine ebenfalls erhöhte Photosyntheserate und damit eine erhöhte Ertragsbildung erwarten.

Als Vergleichssubstanz diente der bekannte Wirkstoff CCC:

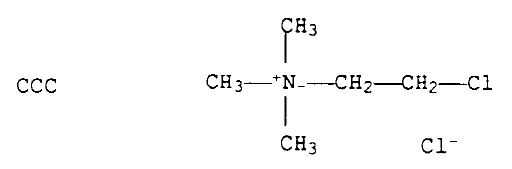

Als Testpflanzen dienten Brassica napus (Winterraps; Abkürzung: BRSNW), Hordeum vulgaris (Sommergerste; Abkürzung: HORVS) und Triticum aestivum (Sommerweizen; Abkürzung: TRZAS). Die Ergebnisse der Versuche sind in den folgenden Tabellen zusammengestellt:

**Tabelle 1**

| | kg/ha a.S. | HORVS^{a)} | TRZAS^{b)} |
|---|---|---|---|
| Substanz | -/- | 100 | 100 |
| CCC | 3 | 83 | 79 |
| | 1,5 | 83 | 84 |
| | 0,75 | 86 | 84 |
| | 0,375 | 92 | 84 |
| Nr. 1 | 0,5 | 61 | 65 |
| | 0,25 | 86 | 81 |
| | 0,125 | 89 | 95 |
| | 0,06 | 92 | 95 |
| Nr. 6 | 0,5 | 41 | 48 |
| | 0,25 | 70 | 56 |
| | 0,125 | 73 | 67 |
| | 0,06 | 83 | 84 |
| Nr. 4 | 0,5 | 89 | 84 |
| | 0,25 | 92 | 90 |
| | 0,125 | 96 | 95 |

| | | | |
|---|---|---|---|
| ^{a)} Sorte "Alexis" | | | |
| ^{b)} Sorte "Star" | | | |

**Tabelle 2**

| | kg/ha a.S. | BRSNW^{a)} |
|---|---|---|
| unbehandelt | - | 100 |
| CCC | 3 | 75 |
| | 1,5 | 85 |
| | 0,75 | 85 |
| | 0,375 | 96 |
| Nr. 6 | 0,5 | 64 |
| | 0,25 | 75 |
| | 0,125 | 85 |
| | 0,06 | 85 |

| | | |
|---|---|---|
| ^{a)} Sorte "Liberator" | | |

## Patentansprüche

1. Piperazin-2,3-dione der Formel I 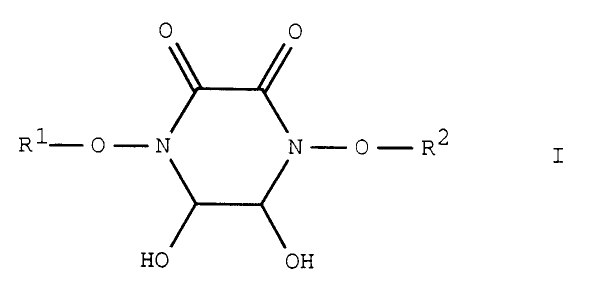 in der die Substituenten folgende Bedeutung haben:
R¹ und R² unabhängig voneinander
C₁-C₂₀-Alkyl, C₃-C₁₈-Alkenyl oder C₃-C₈-Alkinyl, wobei diese Gruppen ein bis fünf Halogenatome tragen können;
C₃-C₁₀-Cycloalkyl, C₅-C₁₀-Cycloalkenyl, C₃-C₁₀-Cycloalkylmethyl, C₅-C₁₀-Cycloalkenylmethyl, Norbornyl, Adamantyl oder Tricyclodecanyl, wobei die Ringe ein bis drei C₁-C₄-Alkylgruppen oder einen Phenylring tragen können;
Phenyl-C₁-C₄-alkyl oder Phenyl-C₂-C₆-alkenyl, wobei die aromatischen Reste ein bis fünf Halogenatome und/oder ein bis drei der folgenden Gruppen tragen können: Nitro, C₁-C₄-Alkyl, C₁-C₄-Halogenalkyl, C₁-C₄-Alkoxy, C₁-C₄-Halogenalkoxy, C₁-C₄-Alkylthio oder C₁-C₄-Halogenalkylthio.

2. Verfahren zur Herstellung der Verbindungen der Formel I gemäß Anspruch 1, dadurch gekennzeichnet, daß man ein Oxalyl-bishydroxamsäurederivat der Formel II,
R¹-ONH-CO-CO-NHOR² II
in an sich bekannter Weise in einem organischem Lösungsmittel oder in Wasser mit wasserfreiem Glyoxal oder mit dessen wäßriger Lösung umsetzt.

3. Mittel zur Regulierung des Pflanzenwachstums, enthaltend eine regulativ wirksame Menge einer Verbindung der Formel I gemäß Anspruch 1 und inerte Zusatzstoffe.

4. Verfahren zur Regulierung des Pflanzenwachstums, dadurch gekennzeichnet, daß man die Pflanzen, ihren Lebensraum und/oder ihre Samen mit einer regulativ wirksamen Menge eines Piperazin-2,3-dions der Formel I gemäß Anspruch 1 behandelt.

5. Verwendung von Piperazin-2,3-dionen der Formel I gemäß Anspruch 1 zur Regulierung des Pflanzenwachstums.

## Claims

1. A piperazine-2,3-dione of the formula I 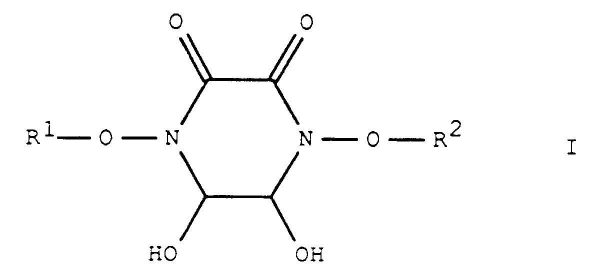 where
R¹ and R² independently of one another are each C₁-C₂₀-alkyl, C₃-C₁₈-alkenyl or C₃-C₈-alkynyl, where these groups may carry from one to five halogen atoms, C₃-C₁₀-cycloalkyl, C₅-C₁₀-cycloalkenyl, C₃-C₁₀-cycloalkylmethyl, C₅-C₁₀-cycloalkenylmethyl, norbornyl, adamantyl or tricyclodecanyl, where the rings may carry from one to three C₁-C₄-alkyl groups or a phenyl ring, or phenyl-C₁-C₄-alkyl or phenyl-C₂-C₆-alkenyl, where the aromatic radicals may carry from one to five halogen atoms and/or from one to three of the following groups: nitro, C₁-C₄-alkyl, C₁-C₄-haloalkyl, C₁-C₄-alkoxy, C₁-C₄-haloalkoxy, C₁-C₄-alkylthio or C₁-C₄-haloalkylthio.

2. A process for the preparation of a compound of the formula I as claimed in claim 1, wherein an oxalylbishydroxamic acid of the formula II
R¹-ONH-CO-CO-NHOR² II
is reacted, in a conventional manner in an organic solvent or in water, with anhydrous glyoxal or with an aqueous solution thereof.

3. A plant growth regulator containing an amount, having a regulating effect, of a compound of the formula I as claimed in claim 1 and inert additives.

4. A method for regulating plant growth, wherein the plants, their habitat or their seed is or are treated with an amount, having a regulating effect, of a piperazine-2,3-dione of the formula I as claimed in claim 1.

5. Use of a piperazine-2,3-dione of the formula I as claimed in claim 1 for regulating plant growth.

## Revendications

1. Pipérazine-2,3-diones de formule I 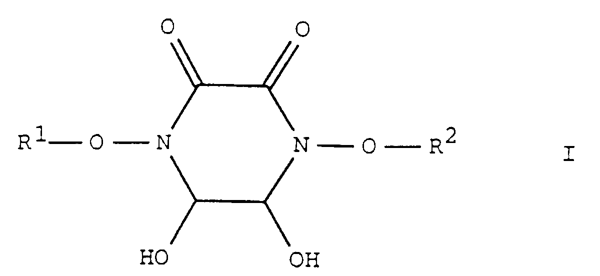 dans laquelle les symboles ont les significations suivantes :
R¹ et R² représentent chacun, indépendamment l'un de l'autre
un groupe alkyle en C1-C20, alcényle en C3-C18 ou alcynyle en C3-C8, ces groupes pouvant porter un à cinq atomes d'halogènes ;
un groupe cycloalkyle en C3-C10, cycloalcényle en C5-C10, (cycloalkyle en C3-C10)méthyle, (cycloalcényle en C5-C10)méthyle, norbornyle, adamantyle ou tricyclodécanyle, les cycles pouvant porter un à trois groupes alkyle en C1-C4 ou un cycle phényle ;
un groupe phényl-alkyle en C1-C4 ou phényl-alcényle en C2-C6, les parties aromatiques pouvant porter un à cinq atomes d'halogènes et/ou un à trois des substituants suivants : nitro, alkyle en C1-C4, halogénoalkyle en C1-C4, alcoxy en C1-C4, halogénoalcoxy en C1-C4, alkylthio en C1-C4 ou halogénoalkyle en C1-C4.

2. Procédé de préparation des composés de formule I de la revendication 1, caractérisé par le fait que l'on fait réagir un dérivé d'acide oxalyl-bis-hydroxamique de formule II
R¹-ONH-CO-CO-NHOR² II
de manière connue en soi, dans un solvant organique ou dans l'eau, avec du glyoxal anhydre ou une solution aqueuse de glyoxal.

3. Produit pour la régulation de la croissance des végétaux, contenant une quantité régulatrice efficace d'un composé de formule I de la revendication 1 et des additifs inertes.

4. Procédé pour la régulation de la croissance des végétaux, caractérisé par le fait que l'on traite les végétaux, leur habitat et/ou leurs semences par une quantité régulatrice efficace d'une pipérazine-2,3-dione de formule I de la revendication 1.

5. Utilisation des pipérazine-2,3-diones de formule I de la revendication 1 pour la régulation de la croissance des végétaux.
